(19) Europäisches Patentamt
European Patent Office
Office européen des brevets

(11) Publication number: 0 349 658
A1

# (12) EUROPEAN PATENT APPLICATION

(21) Application number: 88110466.5

(51) Int. Cl.4: C07C 405/00 , //A61K31/557

(22) Date of filing: 30.06.88

(43) Date of publication of application:
10.01.90 Bulletin 90/02

(84) Designated Contracting States:
BE CH DE FR GB IT LI LU NL SE

(71) Applicant: SPOFA Spojené Podniky Pro Zdravotnickou Vyrobu
Husinecká 11 a
Prag 3(CS)

(72) Inventor: Kral, Josef, Dipl. Ing.
Chotoun 55
Pohori(CS)
Inventor: Picha CSc, Josef, Dipl. Ing.
Za Skolou 469
Jilové u Prahy(CS)
Inventor: Sevcik DrSc, Bohumi, Prof. MVDr
Chotoun 79
Pohori(CS)
Inventor: Bilek, Petr, Dipl. Ing.
Kyjevska 14
Praha 6(CS)

(74) Representative: Patentanwälte Beetz sen. - Beetz jun. Timpe - Siegfried - Schmitt-Fumian
Steinsdorfstrasse 10
D-8000 München 22(DE)

(54) **New prostanoids and their use for increasing the birth rate in animal females.**

(57) The invention relates to novel prostanoid compounds and compositions for increasing the birth rate in female farm animals. The synthetic prostanoids according to the invention are characterized by the general formula I,

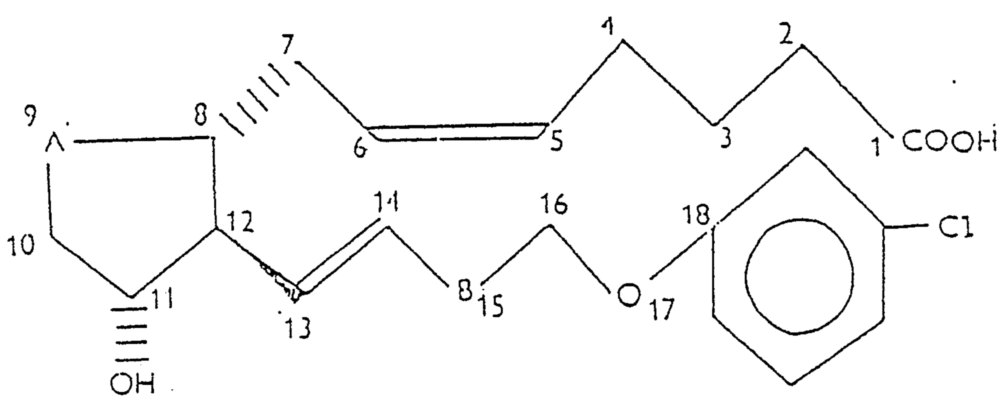

(I),

wherein are:

EP 0 349 658 A1

A 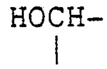 or 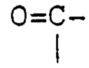 ,

and

B 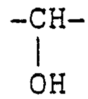 or 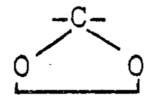 .

These compounds have unexpectedly been found to produce a significant increase in the plasma progesterone level following a single intramuscular injection due to a kind of luteotropic action which is opposite to the known luteolytic action of some structurally related prostaglandins described in the literature. The compounds and compositions are particularly suited for the treatment of cows and sows.

## New prostanoids and their use for increasing the birth rate in animal females

The present invention relates to new synthetically prepared prostanoids of F and E groups and their use for increasing the birth rate in animal females.

All literature data relating to effects of prostanoids F on the function of the corpus luteum describe their luteolytic effects (see e.g. the monographs Schör, K., Prostaglandine und verwandte Verbindungen, Georg Thieme Verlag Stuttgart, New York, 1984, and Zaorská, B., Prostaglandyny i ine eikozanoidy, Panstwowy Zaklad Wydawnictw Lekarskych, Warszawa, 1986). Only one publication mentions a stimulating effect of prostaglandin on the secretion of progesterone. It is the case for the natural prostaglandin $E_2$ (not F). A prolongation of the secretion phase of the corpus luteum following a five-day infusion of prostaglandin $E_2$ into the uterine horn of a cow has been reported (Gimenez, T., Henricks, D.M., Prolongation of the luteal phase by prostaglandin $E_2$, Theriogenology 19 (1983) 693-701). These experiments have been repeated under similar conditions on heifers, however, no stimulating effect on the secretion of progesterone was observed (Schneider, T. M., et al., The effect of intrauterine infusions of prostaglandin $E_2$ on luteal function in nonpregnant gilts, Theriogenology 20 (1983) 509-521).

Tests of biological activities revealed quite unexpectedly that some of new prostanoids tested, and particularly the substance glykal (Staněk, J., et al., Způsob výroby acetalů prostanoidů (The way of production of prostanoid acetals) AO 23065, 1984) increased plasma progesterone levels following a single intramuscular injection. Further experiments demonstrated a stimulating effect on development of secretion cells of the corpus luteum. In comparison with so far known effects of prostanoids on the function of the corpus luteum this is a case of quite contradictory effects. It was expected that the implementation of this new knowledge in practice may bring good results.

Breeding work in cattle aims at the selection of animals for their performance and corresponding external conformation traits. Maximum production and milk fatness were to be achieved as well as fattening capability. However, treatments on that basis produced some unfavourable consequences, particularly in terms of fertility. The resistance of the animals to unfavourable environmental effects was reduced, thus reproduction functions were impaired, which in final consequence lead to a decrease in the conception rate of a herd.

The most sensitive phase of pregnancy is its early stage. The preparation according to this invention can affect just this stage. Under normal circumstances, an ovum is fertilized after insemination, and an early embryo is formed. Implantation of embryos in the uterine wall and its protection against expulsion are achieved by progesterone secreted by the pregnant corpus luteum (CL), a gland on the surface of the ovary. The physiological course of the early pregnancy depends on the function of CL and on a sufficient level of progesterone in the blood plasma. Many herds exhibited a reduced secretion activity of CL, which reflected in fetal mortality and expulsion. The loss by embryonic mortality amounts even in good herds to 15 to 25 % of conceived animals; in herds with reduced fertility it amounts to as much as 40 %. These losses are mainly attributed to insufficient function of CL.

It is the object of the present invention to cope with the above-mentioned disadvantages and drawbacks of the present state and to develop novel synthetic prostanoids which can be used for increasing the birth rate in animal females and particularly farm animals, and to provide pharmaceutical compositions comprising these prostanoids. The above object is achieved according to the claims. The dependent claims relate to preferred embodiments.

The compounds according to the invention are characterized by the general formula I,

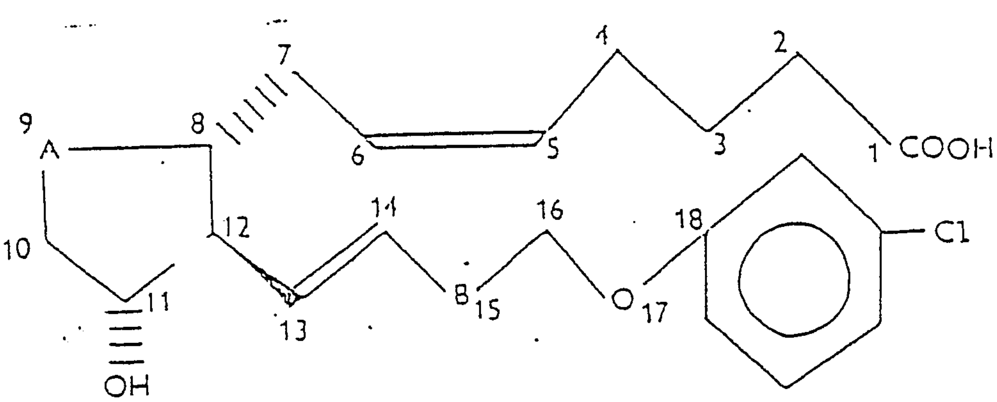

(I),

wherein are:

A HOCH- or O=C-

and

B -CH- or 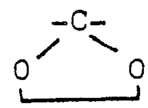 ,
OH

while,

if A is HOCH- then B is 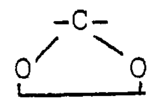 ,

and if A is O=C- then B is -CH- or 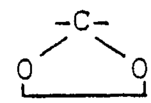 .
OH

The compositions according to the present invention for increasing the birth rate in animal females comprise at least one of these compounds of the general formula I.

The compounds of formula I according to the present invention may be advantageously administered by injection, particularly i.m. injection, whereby a dosage of 0,1 to 1,0 mg per head is used after insemination.

The compounds and compositions according to the invention may be used with particular advantages for the treatment of cows and sows.

The compounds according to the invention exhibit a quite unexpected, markedly pronounced activity in increasing the plasma progesterone level. This result can be obtained by a single intramuscular injection.

The plasma progesterone level increase is attributed to a kind of luteotropic action, which is opposite to the known luteolytic action of some structurally related known prostaglandins.

The optimally suited compound according to the invention is a compound of formula I wherein A is HO-CH- and B is

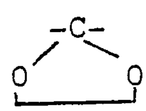

(2,2-connected 1,3-dioxolane residue).

The compounds of formula I can be synthesized according to known methods.

The compounds and compositions according to the present invention can support the development of CL and induce an increase in the progesterone level by 30 to 50 % as compared with control animals.

The pharmacological activity of the compounds of formula I according to the invention was demonstrated in vitro and in vivo on luteal tissue and by practical trials with cows and sows under usual farm animal raising conditions.

1. Study of the effect on luteal tissue in vivo

On day 6 after insemination eight heifers with unaffected function of CL where treated by i.m. injection of the substance of formula I in a dosis of 0,5 mg. The progesterone level in the blood plasma of experimental and 8 comparable control heifers was measured prior to administration and on several following days. The progesterone levels in the blood plasma were increased by 30 to 50 % after administration of the substance of formula I.

2. Study of the effect on luteal tissue in vitro

Luteal tissue was collected from well developed CL of slaughtered cows. The tissue was divided, weighed and put into cultur medium with graded levels of the substance of formula I and into a control medium without active ingredient. The progesterone concentrations were determined in the media at predetermined intervals; the values obtained are calculated as amount of progesterone per 1 mg tissues of CL. The results indicated that all the doses tested prolonged the secretion activity of the luteal tissue, with the medium dose stimulating the secretion for the whole time of the experiment. The results obtained are summarized in Table 1.

3. Study of the effect under conditions of practice in cows

In cows of a herd with low reproduction traits kept in a large cow-house the substance of formula I was used. Cows inseminated in odd weeks were treated, cows inseminated in even weeks were used as a control. 0,5 mg of the compound was injected i.m. into the gluteal muscle after insemination. After 8 months the trial was terminated. The results obtained are summarized in Table 2. The treated cows exhibited a 14,25 % higher conception rate.

4. Study of the effect under conditions of practice in sows

The substance of formula I was tested in a herd of sows with average reproduction traits. On day 3 after A.I. approximately one half of sows were received an i.m. injection of the compound according to the invention at a dose of 0,5 mg. Another half formed a control group. After farrowings the effect of the substance was evaluated according to the number of farrowings in a group and to the number of piglets in a litter. The experimental group indicated better results in both traits studied. The results obtained are summarized in Table 3.

Table 1

| Amount of progesterone (ng) secreted by 1 mg of luteal tissue | | | | | | | |
|---|---|---|---|---|---|---|---|
| | Dose (mg) | 30 min | | 60 min | | 120 min | |
| | | ng | index | ng | index | ng | index |
| Control | 0 | 8,8 | 100 % | 7,8 | 100 % | 2,2 | 100 % |
| Subst. I | 2,5 | 8,4 | 95,4 % | 6,9 | 88,2 % | 4,4 | 200 % |
| -"- | 25 | 10,0 | 113,6 % | 11,2 | 144,1 % | 6,3 | 284,6 % |
| -"- | 250 | 8,6 | 97,7 % | 12,6 | 161,8 % | 4,4 | 200 % |

Table 2

| Percentage of conception rate of cows treated after insemination | | | |
|---|---|---|---|
| Group | Cows inseminated krav | Pregnant | |
| | | heads | (%) |
| Control | 173 | 66 | 38,15 |
| Experimental | 166 | 87 | 52,40 |

Table 3

| Percentage of birth rate and number of piglets in sows | | | | | |
|---|---|---|---|---|---|
| Group | Sows inseminated | Farrowing | | Piglets per litter | Piglets per inseminated sow |
| | | heads | (%) | | |
| Control | 63 | 40 | 63,5 | 9,8 | 6,2 |
| Experimental | 59 | 43 | 72,9 | 11,1 | 8,1 |

The following examples document the useful activity of the compounds and compositions according to the present invention.

**Example 1**

From a herd of heifers individuals are selected convenient for insemination. Of them all-in - all-out groups are formed in which insemination is carried out following a treatment by the luteolytic compound. On day 4 after insemination heifers receive an i.m. injection of 0,25 mg of the substance of formula I. After examination for pregnancy unpregnant heifers are repeadetly inseminated and treated by the substance of formula I as presented above. The experimental group exhibits a 5 to 15 % higher conception rate.

**Example 2**

In a herd of cows inseminations are performed at spontaneous heats. On two working days of a week cows receive on 4 to 6 after insemination an i.m. injection of 1 mg of the substance of formula I at a single dose. After the second and further inseminations of unpregnant cows the substance is adminstered again as described. The experimental group exhibits a 5 to 15 % higher conception rate.

**Example 3**

Mass inseminations of cows treated by the luteolytic compound are performed in a herd. Over 1 to 4 days after insemination the substance of formula I is injected i.m. at a dose of 0,5 mg. The treatments may be repeated after another insemination. The results obtained are summarized in Table 2.

**Example 4**

The compound of formula I is administered at a dose of 0,5 mg by i.m. injection to sows on days 3 or 4

after insemination or mating.

The results obtained are summarized in Table 3 that indicates that administration of the compound according to the invention resulted in an increased number of pregnant animals (increase 9,4 %) and in an increased number of piglets in a litter (increase 1,3 piglets).

## Claims

1. Compounds of the general formula I

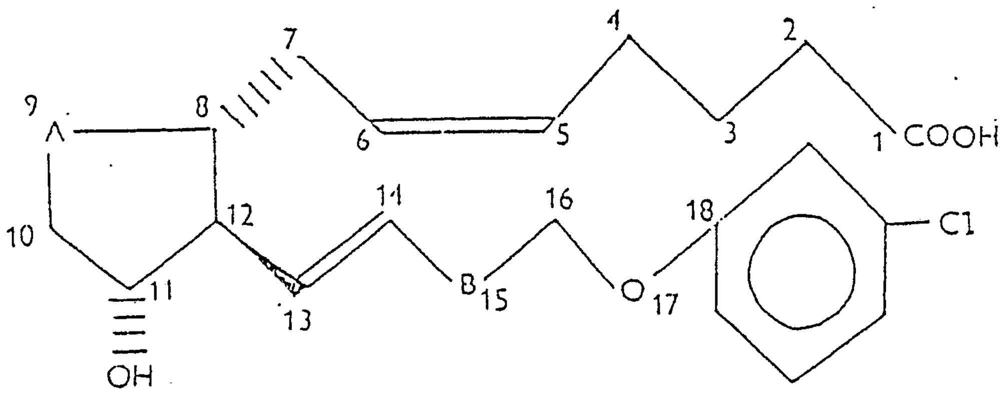

(I),

wherein are:

A HOCH- or O=C-

and

B -CH-
OH

or

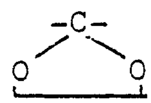

whereby, when

A is HOCH- then B is

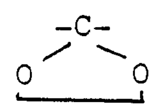

, and,

when A is O=C- then B is -CH-
OH

or

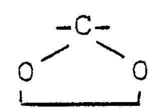

2. Compound of formula I according to claim 1 wherein A is HO- CH- and B is

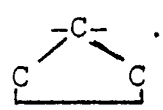

3. Compositions for increasing the birth rate in animal females, characterized by at least one compound of the general formula I according to claims 1 or 2 and usual supports and/or carriers and/or additives.

4. Compositions according to claim 3, characterized in that they are formulated in dosage units of 0,1 to 1,0 mg of compounds of formula I.

5. Compositions according to claim 3 or 4, characterized in that they are formulated in the form of

solutions for injection.

6. Use of the compounds of formula I according to claim 1 or 2 for increasing the birth rate in animal females and particularly farm animals.

7. The use according to claim 5, characterized in that the compounds of formula I according to claim 1 or 2 or the compositions according to one of claims 3 to 5 are administered by i.m. injection of 0,1 to 1,0 mg of the active ingredient of formula I.

European Patent Office

# PARTIAL EUROPEAN SEARCH REPORT

which under Rule 45 of the European Patent Convention shall be considered, for the purposes of subsequent proceedings, as the European search report

Application number
EP 88 11 0466

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | CHEMICAL ABSTRACTS, vol. 106, no. 3, January 19, 1987, page 575, ref.no. 18237z; Columbus, Ohio, US; & CS-A-230 650 (J. STANEK et al.) 15-05-1986 * Abstract * | 1-7 | C 07 C 177/50// A 61 K 31/557 |
| X | FR-A-2 277 577 (SCHERING AKT.) * Page 18, lines 30-32; claims * | 1-7 | |
| X | FR-A-2 184 007 (THE UPJOHN CO.) * Claims * | 1-5 | |
| X | FR-A-2 137 712 (I.C.I.) * Claims * | 1-5 | |

TECHNICAL FIELDS SEARCHED (Int. Cl.4)

A 61 K
C 07 C

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5
Claims searched incompletely: 6,7
Claims not searched:
Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 07-03-1989 | BERTE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document